# EUROPEAN PATENT APPLICATION

(11) **EP 2 196 176 A2**
(43) Date of publication of application: **16.06.2010**
(21) Application number: 08163847.0
(22) Date of filing: 30.09.2003
(51) Int. Cl.: A61F 13/15

(54) **Absorbent article having flaps and stress relief means**

(30) Priority: 07.10.2002 US 265893
(62) Divisional of application: 03808101.4
(71) Applicant: The Procter and Gamble Company, Cincinnati, Ohio 45202 (US)
(72) Inventor: Salone, Fiorello, 65124, Pescara (IT); Lavash, Bruce William, West Chester, OH OH 45069 (US); Bonelli, Guido, 65129, Pescara (IT); Capri, Maria Grazia, 66012, Cepagatti (PE) (IT); Digiacomantonio, Marco, 65125, Pescara (IT)
(74) Representative: L'Huillier, Florent Charles

(57) **Abstract**

An absorbent article, such as a sanitary napkin, having flaps with a combination of first and second stress relief means for relieving the stresses that develop in the flaps when the flaps are folded down along the edges of the wearer's panties in the crotch. The flaps extend laterally outward from the main body portion of the article. The flaps are associated with the main body portion at a juncture along the longitudinal edges of the main body portion. The flaps have a first stress relief means located remote from the juncture. The first stress relief means can be either a slit or a notch. The flaps have at least one second stress relief means contiguous with the first stress relief means. The second stress relief means is a zone of differential extensibility. The zone of differential extensibility can be capable of greater extension in a generally longitudinal direction than other portions of the absorbent article. The zone of differential extensibility can be made of corrugated or ring-rolled portions of the absorbent article.

## Description

### FIELD OF THE INVENTION

The present invention relates to absorbent articles such as sanitary napkins, adult incontinence devices, and the like. Still more particularly, the present invention concerns absorbent articles having flaps with a combination of first and second stress relief means for relieving the stresses that develop in the flaps when the flaps are folded down and under a wearer's undergarment and attached to the underside of the undergarment.

### BACKGROUND OF THE INVENTION

All manner and variety of absorbent articles configured for the absorption of body fluids such as menses, urine, and feces are, of course, well known. Absorbent articles, particularly sanitary napkins, having wings or flaps are disclosed in the literature and are available in the marketplace.

Generally, the flaps extend laterally from a central absorbent means and are intended to be folded around the edges of the wearer's panties in the crotch region. Thus, the flaps are disposed between the edges of the wearer's panties in the crotch region and the wearer's thighs. Commonly, the flaps are provided with an attachment means for affixing the flaps to the underside of the wearer's panties.

The flaps serve at least two purposes. First, the flaps prevent exudates which otherwise would soil the edges of the wearer's panties from doing such. Second, the flaps help stabilize the napkin from shifting out of place, especially when the flaps are affixed to the underside of the panties.

Sanitary napkins having flaps of the various types are disclosed in U.S. Patent 4,687,478, entitled "Shaped Sanitary Napkin With Flaps", which issued to Van Tilburg on August 18, 1987, U.S. Patent 4,608,047, entitled "Sanitary Napkin Attachment Means", which issued to Mattingly on August 26, 1986, U.S. Patent 4,589,876, entitled "Sanitary Napkin", which issued to Van Tilburg on May 20, 1986, U.S. Patent 4,285,343, entitled "Sanitary Napkin", which issued to McNair on August 25, 1981, U.S. Patent 3,397,697, entitled "Disposable Sanitary Shield For Undergarments", which issued to Rickard on August 20, 1968, and U.S. Patent 2,787,271, entitled "Sanitary Napkin", which issued to Clark on April 2, 1957.

While sanitary napkins having flaps are commonly viewed as providing better protection against soiling as compared to sanitary napkins without flaps, the flapped napkins commonly experience problems that keep them from being optimally effective. These problems generally result from the stresses exerted on such flaps when the sanitary napkins are worn.

When the flaps are folded down along the edges of the wearer's panties, stresses are created in the flaps. The stresses are especially high along the fold line at the edges of the wearer's panties where the flaps are bent from the bodyside of the panty to the underside of the panty. These stresses are caused by fitting a flap around the curved outline of a panty crotch. These stresses are magnified when a wearer sits or crouches because the edges of the panties are pulled outward against the flaps thus increasing the forces against this fold line. When the stresses become too high, the flaps may become detached from the panty and some portion of the aforementioned benefits of the flaps may be lost. In addition, even if the stresses are not sufficient to detach the flaps, they may still be sufficient to cause the flaps to bunch longitudinally inward. This effectively reduces the size of the flaps and the area of the wearer's undergarments that the flaps are able to cover. Thus, there is a commercial need for a way of eliminating or at least reducing the stresses that develop in the flaps when folded, so as to prevent them from becoming detached from the wearer's panties and losing the ability to cover a given area of the panties.

A number of variations on the types of flaps described above have been presented in an attempt to solve various problems. U.S. Patent 4,900,320, issued to McCoy on February 13, 1990, discloses a sanitary napkin having flaps affixed at points inward from the longitudinal edge of the napkin. U.S. Patent 4,911,701, issued to Mavinkurve on March 27, 1990, discloses a sanitary napkin having elastic means for providing greater convex shape to the body-facing portion of the central absorbent and for enabling adhesive-free placement of the flaps of the napkin. U.S. Patent 4,940,462, issued to Salerno on July 10, 1990, discloses a sanitary napkin with longitudinally expandable flaps. A sanitary napkin having flaps with stress relief means in the form of a notch or a slit is described in U.S. Patent 4,917,697, which issued to Osborn, III, et al. on April 17, 1990. EP 606 359 B1 discloses a sanitary napkin have flaps with zones of differential extensibility which provide a stress relief means. Although the latter two sanitary napkins works quite well, the search for sanitary napkins having improved flaps has continued.

The stresses described above can also unduly limit the size of the flaps used with an absorbent article since the stresses are typically greater in products having large flaps (that is flaps having a relatively large longitudinal dimension). There is, thus, also a need for an improved stress relief means for relieving the stresses that develop in the flaps, that does not limit the size of the flaps that can be used.

### SUMMARY OF THE INVENTION

In accordance with the present invention, an absorbent article, such as a sanitary napkin, having flaps with a combination of first and second stress relief means for relieving the stresses that develop in the flaps when the flaps are folded down along the edges of the wearer's panties in the crotch, is provided in accordance with the appended claims.

The sanitary napkin has a principal longitudinal centerline and a principal transverse centerline. The sanitary napkin comprises a main body portion and a pair of flaps associated with the main body portion. The main body portion of the sanitary napkin comprises a liquid pervious topsheet, a liquid impervious backsheet joined to the topsheet, and an absorbent core positioned between the topsheet and the backsheet. The main body portion has two spaced apart longitudinal edges and two spaced apart end edges.

The flaps extend laterally outward from the main body portion. The flaps are associated with the main body portion at a juncture along the longitudinal edges of the main body portion. The flaps are divided into a front half and a back half by a flap transverse centerline. The flaps have a first stress relief means located remote from the juncture. The first stress relief means can be either a slit or a notch. The flaps have at least one second stress relief means contiguous with the first stress relief means. The second stress relief means is a zone of differential extensibility. The flap may have a pair of second stress relief means. The pair of second stress relief means may be spaced from one another. The zone of differential extensibility is capable of greater extension in a generally longitudinal direction than other portions of the absorbent article. The zone of differential extensibility is made of corrugated or ring-rolled portions of the absorbent article.

### BRIEF DESCRIPTION OF THE DRAWINGS

Figure 1 is a top plan view of a sanitary napkin embodiment of the present invention.
Figure 1A is a cross-sectional view taken along line 1 A-1 A of Figure 1.
Figure 1B is a cross-sectional view taken along line 1 B-1 B of Figure 1.
Figure 2 is a top plan view of an alternative embodiment of a sanitary napkin of the present invention.
Figure 3 is a perspective view of the crotch portion of a woman's panties.
Figure 4 is a bottom plan view of the sanitary napkin of Figure 1 applied to a woman's panties.

### DETAILED DESCRIPTION OF PREFERRED EMBODIMENTS

All documents cited are, in relevant part, incorporated herein by reference; the citation of any document is not to be construed as an admission that it is prior art with respect to the present invention.

The present invention relates to absorbent articles, such as sanitary napkins. More particularly, the present invention relates to absorbent articles having flaps with a combination of first and second stress relief means for relieving the stresses that develop in the flaps when they are folded down along the edges of the crotch of the wearer's undergarment and attached to the underside of the undergarment.

The term "absorbent article", as used herein, refers to articles which absorb and contain body exudates. More specifically, the term refers to articles which are placed against or in proximity to the body of the wearer to absorb and contain the various exudates discharged from the body. The term "absorbent article" is intended to include sanitary napkins, pantiliners, and incontinent pads (and other articles worn in the crotch region of a garment). The term "disposable" refers to articles which are intended to be discarded after a single use and preferably recycled, composted, or otherwise disposed of in an environmentally compatible manner. (That is, they are not intended to be laundered or otherwise restored or reused as an absorbent article.) In the preferred-embodiment illustrated, the absorbent article is a sanitary napkin designated 20.

The term "sanitary napkin", as used herein, refers to an article which is worn by females adjacent to the pudendal region that is intended to absorb and contain the various exudates which are discharged from the body (e.g., blood, menses, and urine). The present invention, however, is not limited to the particular types or configurations of absorbent articles shown in the drawings.

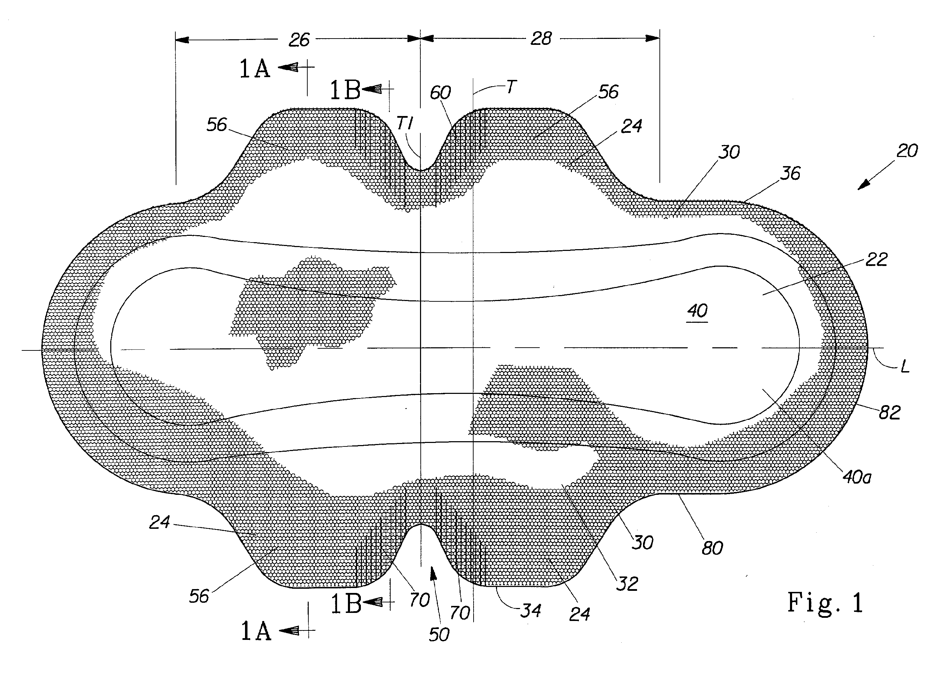

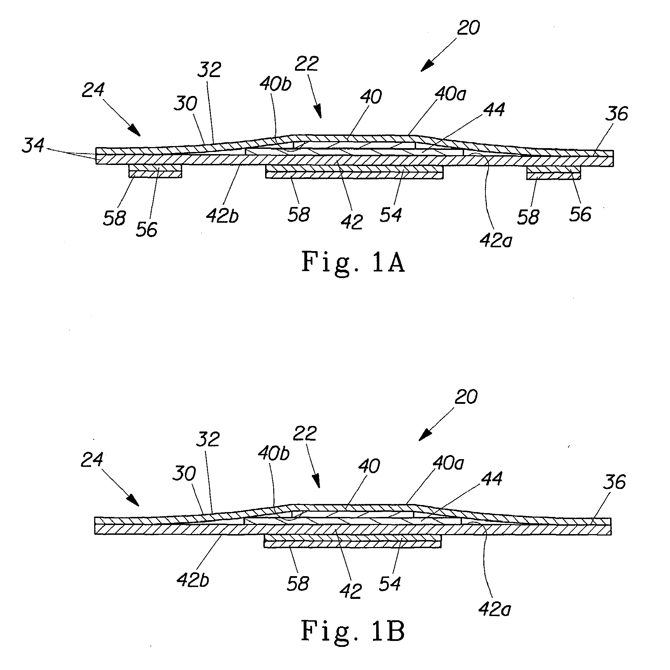

A sanitary napkin 20 according to the present invention is shown in Figure 1. As shown in Figure 1, the sanitary napkin 20 basically comprises an absorbent means represented by central absorbent pad (or "main body portion") 22, and two flaps 24. In the discussion that follows, unless otherwise noted, the sanitary napkin described herein will have two flaps. While it is not necessary that the napkin have two flaps, two flaps are preferred over one flap. Also, while it is not necessary that the flaps be mirror images of one another, they preferably are mirror images of one another. Thus, the description of one flap will be a description of the other, and, for clarity, discussion of the second flap may be omitted.

The sanitary napkin 20 has two centerlines, a principal longitudinal centerline L and a principal transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

Referring now to Figures 1,1A and 1B, the sanitary napkin 20 is comprised of a topsheet 40, a backsheet 42, an absorbent core 44, and a pair of flaps 24. At least a part of the topsheet 40, backsheet 42, and absorbent core 44 comprise the main body portion 22.

The topsheet 40 is liquid permeable and when the sanitary napkin 20 is in use, the topsheet 40 is in close proximity to the skin of the user. The topsheet 40 is compliant, soft feeling, and non-irritating to the user's skin. It can be made from any of the materials conventional for this type of use. Nonlimiting examples of suitable materials that can be used as topsheet 40 are woven and nonwoven polyester, polypropylene, nylon, and rayon and formed thermoplastic films, with formed films being preferred.

Suitable formed films are described in U.S. Patent 3,929,135, entitled "Absorptive Structure Having Tapered Capillaries", which issued to Thompson on December 30, 1975, U.S. Patent 4,324,426, entitled "Disposable Absorbent Article Having A Stain-Resistant Topsheet", which issued to Mullane and Smith on April 13, 1982, U.S. Patent 4,342,314, entitled "Resilient Plastic Web Exhibiting Fiber-Like Properties", which issued to Radel and Thompson on August 3, 1982, and U.S. Patent 4,463,045, entitled "Macroscopically Expanded Three-Dimensional Plastic Web Exhibiting Non-Glossy Visible Surface and Cloth-Like Tactile Impression", which issued to Ahr, Louis, Mullane, and Ouellette on July 31, 1984. Formed films are preferred for topsheet 40 because they are pervious to liquids and yet non-absorbent. Thus, the surface of the formed film which is in contact with the body remains dry and is more comfortable to the wearer.

In addition, in preferred embodiments of the present invention, at least a portion of the outer surface 40a of the topsheet 40 is treated with a surfactant. It is preferred that the surfactant be substantially evenly and completely distributed across at least the portion of the outer surface 40a of topsheet 40 that overlays the main body portion 22. This can be accomplished by any of the common techniques well known to those skilled in the art. For example, the surfactant can be applied to topsheet 40 by spraying, by padding, or by the use of transfer rolls.

Treating the outer surface 40a of the topsheet 40 with a surfactant renders the surface of the topsheet 40 more hydrophilic. This results in liquid penetrating the topsheet 40 faster than it would if the surface were not treated. This diminishes the likelihood that menstrual fluids will flow off topsheet 40 rather than being absorbed by the absorbent core 44. Preferably, any portions of the topsheet 40 that overlay the flaps 24 are not treated with the surfactant. This will minimize any tendencies fluids may have to spread laterally across the flaps and to come in contact with the wearer's thighs and other parts of the wearer's body.

In preferred embodiments, the inner surface 40b of topsheet 40 is secured in contacting relation with the absorbent core 44. This contacting relationship results in liquid penetrating topsheet 40 faster than if the topsheet 40 were not in contact with absorbent core 44. The topsheet 40 can be maintained in contact with absorbent core 44 by applying adhesive to the inner surface 40b of the topsheet 40. Suitable adhesives useful for this purpose are described in U.S. Patent 4,917,697. The adhesives can be applied by the same methods as the surfactant is applied to the outer surface 40a of the topsheet 40.

The absorbent core 44 is positioned between the topsheet 40 and the backsheet 42. The absorbent core 44 provides the means for absorbing menstrual fluid. The absorbent core 44 need not have an absorbent capacity much greater than the total amount of menstrual fluid anticipated to be absorbed. The absorbent core 44 is generally compressible, conformable, and non-irritating to the user's skin. It can comprise any material used in the art for such purpose. Examples include comminuted wood pulp which is generally referred to as airfelt, creped cellulose wadding, absorbent foams, absorbent sponges, synthetic staple fibers, polymeric fibers, hydrogel-forming polymer gelling agents, peat moss, or any equivalent material or combinations of materials.

Polymeric gelling agents are those materials which, upon contact with fluids (i.e., liquids) such as water or body fluids, imbibe such fluids and thereby form hydrogels. In this manner, fluids discharged into the absorbent core 44 can be acquired and held by the polymeric gelling agent, thereby providing the articles herein with enhanced absorbent capacity and/or improved fluid retention performance.

The polymeric gelling agent which is employed in the absorbent core 44 will generally comprise particles of a substantially water-insoluble, slightly cross-linked, partially neutralized, hydrogel-forming polymer material. The term "particles", as used herein, can refer to particles in any form, such as in the form of pellets, flakes, or fibers. The characteristics of the absorbent core 44 (including, but not limited to the preferred types of polymer materials used therein, and types of methods which can be used for preparing these polymer particles) are described in greater detail in U.S. Patent 5,009,653 issued to Osborn and the patents incorporated by reference in that patent, the disclosures of which are all incorporated by reference herein.

In one embodiment, the absorbent core 44 is a laminate comprised of a layer of superabsorbent polymer material, such as in the form of particles, disposed between two air-laid tissues, first and second tissue layers (or "upper" and "lower" tissue layers). The first and second tissue layers provide containment of the superabsorbent polymer material, improve lateral wicking of the absorbed exudates throughout the absorbent core 44 and provide a degree of absorbency.

A suitable laminate is the superabsorbent laminate WATER-LOCK L-535 available from the Grain Processing Corporation of Muscatine, Iowa (WATER-LOCK registered TM by Grain Processing Corporation). Such superabsorbent laminates are disclosed in U.S. Patent 4,467,012 entitled "Composition For Absorbent Film And Method Of Preparation", which issued to Pedersen et al. on August 21, 1984, and U.S. Patent 4,260,443 entitled "Laminated Absorbent Process", which issued to Lindsay et al. on April 7, 1981.

The backsheet 42 is impervious to liquids and, thus, prevents menstrual fluid from soiling the clothing of the user. Any material used in the art for such purpose can be utilized herein. Suitable materials include embossed or nonembossed polyethylene films and laminated tissue. A suitable polyethylene film is manufactured by Monsanto Chemical Corporation and marketed in the trade as Film No. 8020.

In one alternative embodiment of the sanitary napkin 20 (typically in which the topsheet 40 overlays only the main body portion 22 and does not extend out to form the top surface of the flaps), the backsheet 42 may be comprised of two layers. In such a case, the backsheet 42 may comprise a first layer of lofted material disposed on the core-facing side 42a of the backsheet. The purpose of the first layer is to provide a comfortable, non-irritating surface against the body of the wearer. The lofted layer may be comprised of any suitable material, such as a nonwoven material. Preferably, the lofted layer comprises a hydrophobic nonwoven material. The second layer may be disposed on the garment side 42b of the backsheet 42, and may comprise a fluid impervious film. A low density polyethylene material about 0.01 to about 0.05 millimeters in thickness, preferably about 0.02 millimeters in thickness, has been found to work well as this second layer. A polyethylene film, such as is sold by the Ethyl Corporation, Visqueen Division, under model XP-39385 has been found particularly well suited for this second layer. The backsheet 42 may also be made of a soft, cloth-like material which is hydrophobic relative to 'the topsheet 40. A polyester or polyolefinic fiber backsheet 42 has been found to work well. A particularly preferred soft, cloth-like backsheet 42 material is a laminate of a polyester nonwoven material and a film such as described in U.S. Patent 4,476,180 issued to Wnuk on October 9, 1984.

The topsheet 40 is joined or secured to backsheet 42 along a seam 36. The seam 36 can be formed by any means commonly used in the art for this purpose such as by gluing, crimping, or heat-sealing. The topsheet 40 may also be indirectly joined to the backsheet 42.

The main body portion 22 is the portion of the sanitary napkin 20 that contains an absorbent means, such as absorbent core 44. The main body portion 22 has a liquid pervious body contacting surface and an opposed liquid impervious surface. It is to be understood that the embodiment illustrated is only one possible embodiment, albeit a preferred one. Other possible embodiments include one in which an absorbent core 44 is essentially completely wrapped with topsheet before it is placed on a backsheet. The main body portion 22 can also comprise an absorbent core which possesses sufficient integrity to stand alone and is liquid pervious on one surface while the other surface has been treated to render it liquid impervious.

The main body portion 22 may be relatively thick or relatively narrow and thin. A narrow main body portion 22 may be effective because the overall configuration and use of sanitary napkin 20 results in main body portion 22 being maintained in close proximity to the body. Such proximity of main body portion 22 places it precisely where it should be: very near the body at the vaginal opening. The main body portion 22 can then absorb the vast majority of the menstrual fluid (menses) before it has an opportunity to flow along the sides of the main body portion 22. A thin main body portion may also be desired because it is typically comfortable to the user.

Fasteners, such as adhesive attachment means, central pad adhesive 54 and flap adhesive 56, are provided to secure the sanitary napkin 20 to the crotch region of an undergarment.

The central pad adhesive 54 provides an adhesive attachment means for securing main body portion 22 in the crotch portion of a panty. The outer surface of flap 24, adjacent the distal edge 34 of the flap, is preferably coated with a flap adhesive 56. The flap adhesive 56 is used to assist in maintaining the flap 24 in position after it is wrapped around the edge of the crotch portion of the panty as described below. The flaps 24 can be maintained in position by attaching the flaps 24 to the undergarment, or to the opposing flap. Suitable adhesive fasteners are described in greater detail in U.S. Patent 4,917,697.

As shown, each flap 24 comprises a pair of flap adhesives. One flap adhesive 56 is positioned in the front half 26 of flap 24 while the other flap adhesive 56 is positioned on the back half 28 of flap 24. While a pair of flap adhesives 56, one positioned in the front half 24 and the other positioned in the back half 28 are preferred, other embodiments, having a single flap adhesive 56 may also be used. Other embodiments having three or more flap adhesives 56 may also be used.

The fasteners used with the present invention are not limited to adhesive attachment means. Any type of fastener used in the art can be used for such purpose. For example, the sanitary napkin 20 could be secured to the wearer's undergarment by the fastener described in U.S. Patent 4,946,527 entitled "Pressure-Sensitive Adhesive Fastener and Method of Making the Same" issued to Battrell on August 7, 1990. For simplicity, however, the fasteners will be described in terms of adhesive attachment means.

The adhesive attachment means are respectively covered by removable release liners, central pad release liner and flap release liner, both designated 58. The pressure-sensitive adhesives should be covered with release liners 58 to keep the adhesives from sticking to extraneous surfaces prior to use. Suitable release liners are described in U.S. Patent 4,917,697.

The flaps 24 shown are integral with the main body portion 22. In such a case, the topsheet 40 forms one surface of both the flaps 24 and the main body portion 22, and the backsheet 42 may form the other surface of the same. In addition, the absorbent material of the sanitary napkin 20 may extend into the flaps 24 to form a flap absorbent core, as described in greater detail in U.S. Patent 4,917,697. Alternatively, the flaps may be comprised of separate pieces of material which are attached to the main body portion 22, as described in EP 606 358 B1.

The flaps 24 are each associated with main body portion 22 along a juncture. This is typically a longitudinally-oriented (or "longitudinal") juncture, such as lines of juncture 30. As used herein, the terms "juncture" (or "line of juncture") refer to regions where the flaps 24 extend from or are joined to the main body portion 22. These regions can be any of various curved or straight lines, but they are not limited to lines. Thus, these regions can comprise flanges, strips, intermittent lines, and the like. In the embodiment illustrated in Figure 1, line of juncture 30 is a curved line.

The flaps 24 have a proximal edge 32 adjacent the line of juncture 30. A distal edge (or "free end") 34 is remote from the line of juncture 30. As shown in Figure 1, each flap 24 is divided into a front half 26, and a back half 28 by a flap transverse centerline T1. The flap transverse centerline T1 may coincide with the principal transverse centerline T of the sanitary napkin, but this is not absolutely required. The flap transverse centerline T1 extends through the principal longitudinal centerline L.

The overall size of the flaps 24 can be readily selected by those skilled in the art. Preferably, the flaps 24 are sized so that the sanitary napkin 20 is from about 10 to about 23 centimeters wide between the distal edges 34 of the flaps at their greatest separation. Preferably each flap 24 is from about 5 to at least about 19 centimeters long in the direction parallel to the principal longitudinal centerline L of the sanitary napkin.

The shape of the flaps 24 can be selected by those skilled in the art. Preferably, not only are the flaps 24 mirror images of each other, the two halves of each flap 26 and 28 are also symmetrical about the flap transverse centerline T1. (It should be understood that the shape and orientation of the flaps described herein are those of a preferred embodiment. They are not mandatory design features.)

In the embodiment illustrated in Figure 1, the flaps 24 are positioned slightly forward of the principal transverse centerline T of the sanitary napkin. (In such a case, the flap transverse centerline T1 does not coincide with the principal transverse centerline T of the sanitary napkin 20.) The flaps 24, however, are preferably evenly spaced from the principal longitudinal centerline T of the sanitary napkin.

The flaps 24 can be associated with the main body portion 22 in a number of different manners. Many of the different ways a component (such as the flaps 24) can be "joined to" or "associated with", etc. another component which are set forth in the definitions of these terms contained in U.S. Patent 5,007,906 entitled "Decoupled Sanitary Napkin" which issued to Osborn, et al. on April 16, 1991. When the flaps comprise separate elements, they can be joined to the main body portion 22 by any techniques known to those skilled in the art. Such techniques include, but are not limited to adhesives, heat and/or pressure, ultrasonics, etc.

The flaps 24 are associated with the main body portion 22 along lines of juncture 30. The lines of juncture can be concave, straight, (or, but preferably not convex) relative to the principal longitudinal centerline L. The lines of juncture 30 may comprise those lines or areas where separate flap elements are joined to the main body portion 24. Alternatively, when the flaps 24 are integral with the main body portion 22, the lines of juncture 30 may represent lines of demarcation between the main body portion 22 and the flaps 24 (although it is not necessary that there be a precise line of demarcation).

It is also not necessary that the flaps 24 extend from (or be joined along) the longitudinal edges 22 of the main body portion 22. The flaps 24 can be joined inward (or "inboard") from the longitudinal edges 80 toward the longitudinal centerline such as is shown in U.S. Patent 4,900,320 issued to McCoy on February 13, 1990. The flaps 24 can, thus, each be joined to the main body portion 22 along the principal longitudinal centerline L, or along the longitudinal edges 80 of the main body portion 22, or at any place between the principal longitudinal centerline L and the longitudinal edges 80 of the main body portion 22. The flaps 24 will, of course, generally be on opposite sides of the principal longitudinal centerline L.

The flaps 24 have a first stress relief means 50 and a second stress relief means 70. The first stress relief means 50 is positioned remote from the line of juncture 30. In the embodiment shown in Figure 1, the first stress relief means 50 is positioned along the distal edge 34 and centered on the flap transverse centerline T1. The first and second stress relief means relieve the stresses that develop in the flaps 24 when they are folded around a panty crotch.

The first stress relief means 50 may be either a slit or a notch. As used herein, the term "notch" refers to a space, indentation, or hollow along the edge of a material or a laminate of materials. As used herein the term "slit" refers to a narrow cut wherein two edges of material are adjacent or nearly adjacent one another without being joined to one another. A slit may be either linear or curvilinear.

The second stress relief means 70 is contiguous with the first stress relief means 50. In the embodiment shown in Figure 1, the flaps 24 each have two second stress relief means 70 contiguous with the first stress relief means 50. The two stress relief means 70 are spaced from one another on opposite sides of the flap transverse centerline T1. While a pair of second stress relief means 70 contiguous with the first stress relief means 50 is preferred, other embodiments, having a single second stress relief means 70 contiguous with the first stress relief means 50 may also be used. Other embodiments having three or more second stress relief means 70 contiguous with the first stress relief means 50 may also be used.

In the embodiment shown in Figure 1, a second stress relief means 70 is positioned on either side of the flap transverse centerline T1. Thus one second stress relief means 70 resides on the front half 26 of flap 24 while the other second stress relief means 70 resides on the back half 28 of flap 24.

The second stress relief means 70 comprises a zone of differential extensibility. The term "zone of differential extensibility", as used herein, refers to a portion of the sanitary napkin 20 that is capable of extending a differing amount (preferably a greater amount), than surrounding portions of the sanitary napkin 20. The zones of differential extensibility of the second stress relief means 70 are preferably primarily extensible in a greater amount generally in the longitudinal direction. As used herein, "generally in the longitudinal direction" means that the extensibility has a longitudinal component. All of the extension, however, need not be exactly parallel to the principal longitudinal centerline of the sanitary napkin. The extensibility is preferably oriented more in the longitudinal direction than in the transverse direction.

The differential extensibility referred to herein preferably elasticless. That is, it is accomplished without the use of separate elastic pieces, strands, or materials to contract one or more portions of the sanitary napkin. Suitable structures for the zones of differential extensibility are zones of material that are corrugated or ring rolled.

Suitable processes for ring rolling or corrugating are described in U.S. Patent 4,107,364 issued to Sisson on August 15, 1978, U.S. Patent 4,834,741 issued to Sabee on May 30, 1989, U.S. Patent 5,167,897 issued to Weber et al. on December 1,1992, U.S. Patent 5,156,793 issued to Buell et al. on October 20, 1992, and U.S. Patent 5,143,679 issued to Weber et al. on September 1, 1992.

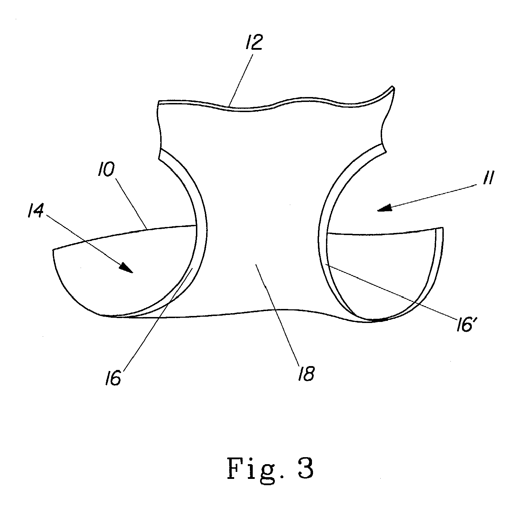

Figure 3 is a depiction of the crotch portion 14 of an undergarment 11 of the type commonly worn by many women and well known as a panty. A panty 11 comprises a front section 10, a back section 12, and a crotch portion 14 which joins the front and back sections. The crotch portion 14 comprises two side edges 16 and center crotch portion 18.

The sanitary napkin 20 of the present invention is utilized by removing the release liners 58 and placing the sanitary napkin 20 in a panty 11. The center of main body portion 22 is placed in crotch portion 14 of the panty with one end of main body portion 22 extending towards the front section 10 of the panty and the other end towards the back section 12. The backsheet 42 is placed in contact with the inner surface of center crotch portion 18 of the panty. Central pad adhesive 54 maintains the main body portion 22 in position. The distal portions of flaps 24 are folded around the side edges 16 of the panty. The flap adhesives secure the flaps 24 to the underside of the panty.

When the flaps 24 are folded down around the edge 16 of the crotch portion 14 of the panty, stresses are developed in the flaps. These stresses are magnified when the flaps 24 are folded under the panty and attached to the panty's underside. The stresses are further magnified when the panty is pulled up into position and the elastics in the panty edges 16 force the folded portion of the flaps into the uppermost part of the wearer's crotch and thigh.

When the flap 24 changes from being disposed on the bodyside of the panty to being located on the underside of the panty stresses develop in the flap as the flap attempts to follow the arc formed by the edges 16 of the crotch portion 14. These stresses cause the flaps 24 to bunch longitudinally inward. This bunching reduces the area of the wearer's panty the flaps are able to cover and creates a less than optimal fit of the flap to the panty.

In order to better approximate the arc of the panty and provide better overall fit, the flaps are provided with a combination of first and second stress relief means for relieving the stresses that develop in the flaps when the flaps are folded down and under a wearer's undergarment and attached to the underside of the undergarment. In order to provide better overall fit, the combination of stress relief means are located remote from the line of juncture 30. The combination of stress relief means allows the flap to better follow the arc of the panty to such a degree that the flaps 24 will not bunch and will provide more coverage of the wearer's panty.

The first stress relief means 50 and the second stress relief means 70 are located in the flaps 24 remote from the line of juncture 30. As mentioned earlier, the first stress relief means 50 may be either a slit or a notch. The slit or notch may be of any shape. The overall dimension of the first stress relief means 50 can vary widely. Preferably, the first stress relief means 50 is not so large that it extends to the line of juncture 30. A balance must be reached between the desired area coverage for the panty and the stress relief being sought when selecting the overall dimension of the first stress relief means 50.

The second stress relief means 70 is contiguous with the first stress relief means 50. The second stress relief means 70 comprises a zone of differential extensibility. The zone of differential extensibility may be of any shape. Typically, it will form a three-sided figure (roughly triangular, pie-shaped, or fan-shaped) in plan view when fully extended. It should be understood, however, that the precise shape of the zone of differential extensibility is not always as critical as the location and extensibility properties of the same. Likewise, it is not critical for there to be precise lines of demarcation that marks the boundaries of the zones of differential extensibility. Thus, there can be a gradual transition between the zones of differential extensibility and the remainder of the flap.

The zones of differential extensibility are typically bounded on one side by at least a portion of the distal edge 34 of the flap 24. This is often a curved line. The remaining sides of the zones of differential extensibility are typically found within the remainder of the flap 24 and do not extend into the main body portion 22.

The total area covered by the zones of differential extensibility can vary widely. The area can cover a relatively large portion of the flap 24, provided there remains some portions of the flap 24 that are less extensible.

The amount of differential extensibility needed can vary depending on a number of factors. These include, but are not limited to the size and configuration of the wearer's panties, the size and configuration of the flaps, etc. Any amount of differential extensibility provided in combination with the first stress relief means will provide some benefit versus a sanitary napkin that is not provided with the combination. The amount of differential extensibility should not be so great, however, that the excess material that comprises the zones of differential extensibility causes the sanitary napkin to fit sloppily to the wearer's panties.

The stress relief means 70 have been provided with differential extensibility by ring rolling these regions in accordance with the earlier-described ring rolling patents. The ring rolling or corrugating should be applied so that the fold lines 60 in the corrugations are oriented generally in the transverse direction. The phrase "generally in the transverse direction" (and similar phrases), as used herein, means oriented more in the transverse dimension than in the longitudinal dimension. Thus, the fold lines 60 may angle away from the principal transverse centerline T. This will provide the desired longitudinal direction extensibility.

The amount of extensibility provided can be varied throughout different portions of the zones of differential extensibility. For instance, the number or amplitude of the corrugations formed by the ring rolling could be varied so that either or both these characteristics are greater closer to the flap transverse centerline T1. This will allow the sanitary napkin to be provided with differential extensibility characteristics that most closely match the configuration of a panty crotch.

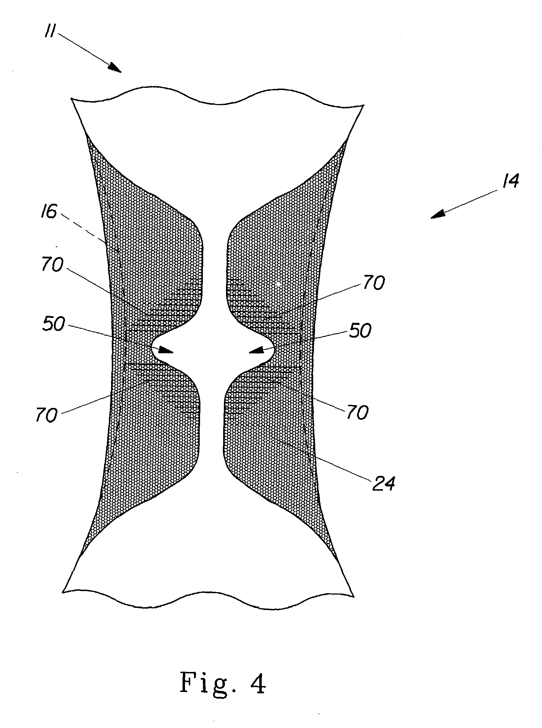

Preferably, the amount of differential extensibility is sufficient to substantially reduce the stresses on the flaps allowing the main body portion and flaps to reside flat against the panty when the sanitary napkin is worn. Figure 4, shows the sanitary napkin of Figure 1 attached to a panty 11. As can be seen the zone of differential extensibility is fully extended allowing the flap 24 to lie flat against the panty. Furthermore, the flap is able to follow the arc formed by the edges 16 of the crotch portion 14 of the panty 11.

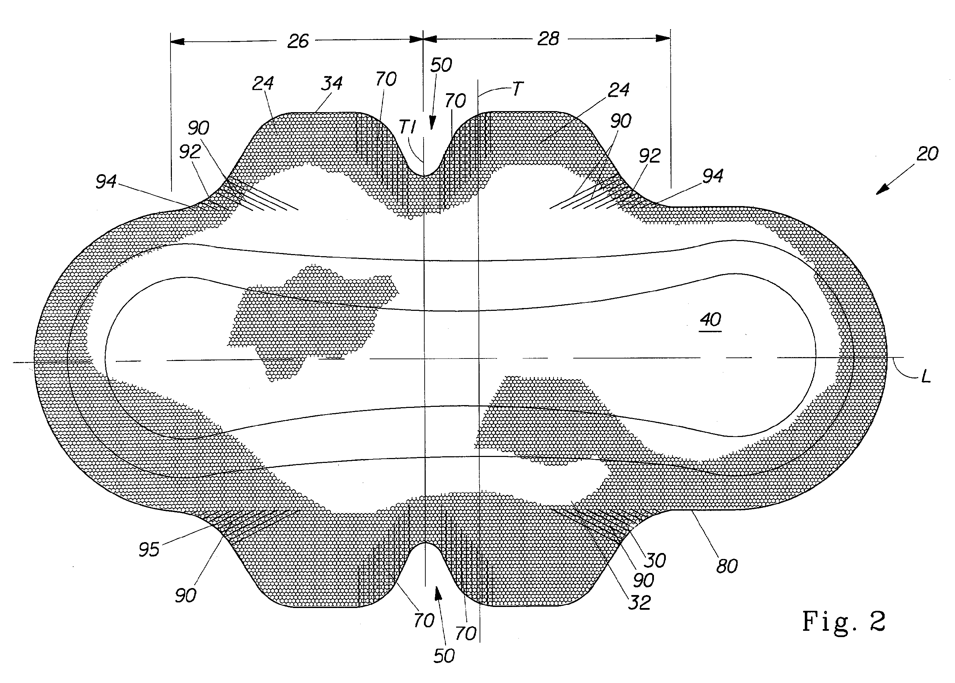

Figure 2 is an alternative embodiment of a sanitary napkin of the present invention having additional zones of differential extensibility 90. The zones of differential extensibility 90 are preferably primarily extensible in a greater amount generally outward in the transverse direction. This is generally in the direction of the arrows shown in Figure 2. As used herein, "generally in the transverse direction" means that the extensibility has a transverse component. All of the extension, however, need not be exactly parallel to the principal transverse centerline of the sanitary napkin. The extensibility is preferably oriented more in the transverse direction than in the longitudinal direction.

The zones of differential extensibility 90 can comprise portions of the main body portion 22, portions of the flaps 24, or both.

The zones of differential extensibility 90 are more specifically located in the corner regions 92 of the sanitary napkin 20. The sanitary napkin 20 preferably has four corner regions 92, two by each flap 24. The term "corner regions" 92, as used herein, refers to portions of the sanitary napkin 20 that are generally located along or adjacent a portion of the longitudinal juncture of each flap 24. The corner regions 92 for each flap 24 are located in two areas in the regions of the ends 94 of each juncture 30. One corner region 92 is located adjacent the longitudinal juncture 30 in the front half 26 of the flap 24. The other is adjacent the longitudinal juncture 30 in the back half 28 of the flap 24. The corner regions 92 are preferably at least partially disposed longitudinally away from the flap transverse centerline T1 in each direction. (Thus, the corner regions 92 may be described as being longitudinally "remote" from the flap transverse centerline T1.)

In the most preferred case (as will be subsequently described in greater detail), the zones of differential extensibility 90 are located along a portion of the fold line where the flaps 24 are folded around the wearer's panty crotch. The fold line will typically be located along or adjacent the longitudinal juncture 30 of each flap 24. Since the terms "portions", "zones", and "regions", as used herein, refer to general areas, the zones of differential extensibility 90 and the corner regions 92 are, thus, not limited to points which lie precisely on the lines of juncture 30. Typically, they will include both those points which lie on the lines of juncture 30 as well as the surrounding areas of the sanitary napkin 20 (which include the aforementioned fold lines). The longitudinal junctures, thus, typically serve as good approximations for the location of the zones of differential extensibility 90.

The corner regions 92 are designated as such because they typically include the "corners" formed along the periphery of the sanitary napkin 20. The "corners" occur where the edges 95 of the flaps 24 intersect with the longitudinal side edges 80 of the main body portion 22 when the sanitary napkin 20 is shown in a plan view. It is not necessary for there to be a sharp angle formed at the intersection of these edges, or for lines of demarcation to designate the same, however.

The zones of differential extensibility 90 may be of any shape. Typically, they will form a three-sided figure (roughly triangular, pie-shaped, or fan-shaped) in plan view when they are fully extended. Often, the figure defined by the zones of differential extensibility will have two sides that are of approximately equal length and a shorter side. The edge 35 of the flaps 24 usually forms the shorter side. It should be understood, however, that the precise shape of the zones of differential extensibility 90 is not always as critical as the location and extensibility properties of the same. Likewise, it is not critical for there to be precise line of demarcation that marks the boundaries of the zones of differential extensibility 90. Thus, there can be a gradual transition between the zones of differential extensibility 90 and the other portions of the sanitary napkin.

The zones of differential extensibility 90 may be bounded on one side by the line of juncture 30. Alternatively, the boundary may be adjacent the line of juncture 30. If the zones of differential extensibility 90 are provided in the main body portion 22 (for instance, if they are formed by a fold made through the main body portion 22, however, this boundary may be as far inboard as the principal longitudinal centerline L. The zones of differential extensibility 90 are typically bounded at the ends by at least a portion of the edge 35 of the flap 24. This is often a curved line. The zones of differential extensibility 90 can also be bounded at the ends by a portion of the longitudinal edges 80 of the main body portion and/or end edges 82 of the main body portion 22. The third side of the zones of differential extensibility is typically formed by a boundary which may be an imaginary line that runs from the point of the zone of differential extensibility 90 which is either located on the flap transverse centerline T1 (or nearest to the same), to a point on the edge 35 of the flap 24.

The total area covered by the zones of differential extensibility 90 can vary widely. The area can cover a relatively large portion of the sanitary napkin, provided there remain some portions of the sanitary napkin adjacent at least portions of the principal longitudinal centerline and the flap transverse centerline that are less extensible. The zones of differential extensibility 90 can be provided along the entire juncture 30 of the flaps 24 with the main body portion 22. Preferably, in the present invention, zones of differential extensibility 90 are not provided either along the entire juncture 30 or throughout the entire flap.

The amount of differential extensibility needed can vary depending on a number of factors. These include, but are not limited to the size and configuration of the wearer's panties, the size and configuration of the flaps, etc. Any amount of differential extensibility in the corner regions 92 will provide some benefit versus a sanitary napkin that is not provided with zones of differential extensibility. The amount of differential extensibility should not be so great, however, that the excess material that comprises the zones of differential extensibility 90 causes the sanitary napkin to fit sloppily adjacent the wearer's panties and her body.

Thus, the present invention provides a sanitary napkin having flaps and zones of differential extensibility to provide an improved stress relief means for relieving the stresses that develop in the flaps when the flaps are folded down and under a wearer's undergarment.

While particular embodiments of the present invention have been illustrated and described, it would be obvious to those skilled in the art that various other changes and modifications can be made without departing from the spirit and scope of the invention.

## Claims

1. An absorbent article (20) for wearing in an undergarment (11), said absorbent article (20) having a principle longitudinal centerline (L) and a transverse centerline (T), said absorbent article (20) comprising:
a main body portion (22) having two spaced apart longitudinal side edges (80) and two spaced apart transverse end edges (82), said main body portion comprising a liquid pervious top sheet (40), a liquid impervious backsheet (42) joined to said topsheet (40) and an absorbent core (44) positioned between said topsheet (40) and said backsheet (42);
a pair of flaps (24) for folding around and securing said absorbent article (20) to said undergarment (11), said flaps (24) being associated with said main body portion (22) at a juncture (30), with one flap (24) extending laterally outward from each longitudinal side edge (80) of said main body portion (22); **characterized in that**
a first stress relief means (50) is located in said flaps (24) remote from said juncture (30), said first stress relief means (50) comprising a slit or a notch; and
at least one second stress relief means (70) is contiguous with said first stress relief means (50) said second stress relief means (70) comprising a zone of differential extensibility.

2. The absorbent article of claim 1 wherein said flap comprises a pair of second stress relief means (70).

3. The absorbent article of claim 2 wherein said pair of second stress relief means (70) are spaced from one another.

4. The absorbent article of any one of the preceding claims wherein said zone of differential extensibility is capable of greater extension in a generally longitudinal direction than other portions of said absorbent article.

5. The absorbent article of any one of the preceding claims wherein said zone of differential extensibility is made pre-corrugated or ring rolled portions of said flap (24).

6. The absorbent article of any one of the preceding claims further comprising a third stress relief means (90) located adjacent said juncture (30), said third stress relief means (90) comprising a zone of differential extensibility.

7. The absorbent article of claim 6 wherein said zone of differential extensibility is capable of greater extension in a generally transverse direction than other portions of said absorbent article.

8. The absorbent article of claim 6 wherein said zone of differential extensibility is made of corrugated or ring rolled portions of said absorbent article (20).
